# EUROPÄISCHE PATENTSCHRIFT

(11) **EP 1 204 946 B1**
(45) Veröffentlichungstag und Bekanntmachung des Hinweises auf die Patenterteilung: **11.06.2003**
(21) Anmeldenummer: 00958203.2
(22) Anmeldetag: 02.08.2000
(51) Int. Cl.: G06K 9/00

(54) **KRATZFESTE BESCHICHTUNG FÜR HALBLEITERBAUELEMENTE**
SCRATCHPROOF COATING FOR SEMICONDUCTOR COMPONENTS
RECOUVREMENT RESISTANT A L'ABRASION POUR COMPOSANTS A SEMI-CONDUCTEUR

(30) Priorität: 02.08.1999 DE 19936322
(43) Veröffentlichungstag der Anmeldung: 15.05.2002
(73) Patentinhaber: Infineon Technologies AG, 81669 München (DE)
(72) Erfinder: FRANK, Manfred, D-93152 Nittendorf (DE); KRÖNINGER, Werner, D-93073 Neutraubling (DE); KÖPNICK, Renate, D-93053 Regensburg (DE); HUMMEL, Richard, D-93133 Burglengenfeld (DE); FISCHBACH, Reinhard, D-81829 München (DE); OPOLKA, Heinz, D-93059 Regensburg (DE)
(74) Vertreter: Epping Hermann & Fischer
(86) Internationale Anmeldenummer: DE0002568
(87) Internationale Veröffentlichungsnummer: WO01009819

(56) Entgegenhaltungen:
- EP-A- 0 791 899
- EP-A- 0 915 373
- US-A- 5 479 049

## Beschreibung

Die vorliegende Erfindung betrifft eine kratzfeste Beschichtung für Halbleiterbauelemente, die insbesondere als Auflagefläche für Fingerabdruckmessung geeignet ist.

Halbleiterbauelemente, die Umwelteinflüssen und insbesondere mechanischem Verschleiß ausgesetzt sind, benötigen eine besonders harte und kratzfeste Passivierung. Insbesondere bei Fingerabdrucksensoren tritt das Problem auf, daß die für die Auflage einer Fingerbire vorgesehene Auflagefläche mechanischem Verschleiß ausgesetzt ist, der die Eigenschaften des Fingerabdrucksensors wesentlich verschlechtert. Bei Fingerabdrucksensoren, die nach dem kapazitiven Meßverfahren arbeiten, kommt es darauf an, daß der Abstand zwischen einer aufliegenden Fingerbire und Leiterebenen in dem Halbleiterbauelement des Sensors auch nach längerem Gebrauch des Sensors innerhalb enger Toleranzen konstant gehalten wird. Herkömmliche Passivierungsschichten aus Siliziumoxid oder Siliziumnitrid, wie sie üblicherweise in der Halbleitertechnologie verwendet werden, reichen bei einer stärkeren Beanspruchung der Oberfläche der Bauelemente nicht aus. Die Verwendung dickerer Passivierungsschichten oder üblicher Passivierungsmaterialien wie zum Beispiel Polyimid reichen nicht aus, da durch dickere Passivierungen die Empfindlichkeit des Sensors herabgesetzt wird.

Die Erprobung von Fingerabdrucksensoren, die mit üblichen Passivierungsschichten passiviert wurden, hat gezeigt, daß vor allem eine hohe Kratzfestigkeit der Fingerauflagefläche derartiger Sensoren erforderlich ist.

Dokument EP 0 791 899 A beschreibt einen Fingerabdruckmessgerät mit einem entsprechenden Fingerabdrucksensor, der eine dünne Siliziumnitridbeschichtung mit einer äußersten, ebenfalls dünnen Polytetrafluoräthylen oder Polyimid-Beschichtung aufweist, um die Verschleißfestigkeit zu erhöhen bzw. eine physikalischen Schutz des Sensors auch gegen Abrasion zu bilden.

Dokument US 5 479 049 A beschreibt eine transparente Schutzschicht auf Colorfiltern und Mikrolinsen mit einem Perfluoralkylpolyether als äußerster Schicht. Diese einem anderen Zweck dienende Schutzschicht wird z.B. auf CCD-Flächensensoren aufgebracht.

Aufgabe der vorliegenden Erfindung ist es, eine Beschichtung für Halbleiterbauelemente anzugeben, die auch bei geringer Schichtdicke und hoher Beanspruchung ausreichend hart und kratzfest ist, um insbesondere auch bei Fingerabdrucksensoren eingesetzt werden zu können.

Diese Aufgabe wird mit der Beschichtung mit den Merkmalen der Ansprüche 1 und 4 gelöst. Ausgestaltungen ergeben sich aus den abhängigen Ansprüchen, 2-3 und 5.

Der erfindungsgemäßen Beschichtung liegt die Erkenntnis zugrunde, daß Kratzer auf einer Passivierungsschicht vor allem durch Scherkräfte, die auf die Oberfläche wirken, verursacht werden. Die erfindungsgemäße Beschichtung ist eine Gleitschicht, die die Eigenschaft hat, derartige Scherkräfte soweit zu verringern, daß eine Beschädigung der Oberfläche verhindert und die Abnutzung bei bestimmungsgemäßem Gebrauch stark reduziert wird. Da diese Gleitschicht sehr dünn aufgebracht werden kann, wird bei deren Verwendung die Empfindlichkeit des kapazitiv messenden Sensors nicht herabgesetzt. Diese Gleitschicht wird vorzugsweise auf eine übliche Passivierungsschicht aus einem Oxid und/oder einem Nitrid aufgebracht.

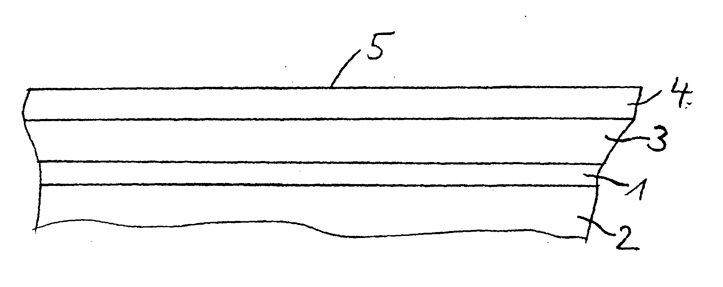

In der beigefügten Figur ist ein typischer Schichtaufbau eines kapazitiv messenden Sensors auf Halbleitermaterial im Querschnitt dargestellt.

Eine Leiterschicht 1, die zum Beispiel bei einem Fingerabdrucksensor in eine Vielzahl rasterförmig angeordneter Leiterflächen, die Bildpunkten entsprechen, aufgeteilt sein kann, befindet sich an der Oberseite eines Halbleiterbauelementes 2, das in der Figur ohne Struktur nur im Ausschnitt angedeutet ist. Die Leiterschicht 1 ist mit einer Passivierungsschicht 3 bedeckt, die zum Beispiel Polyimid oder eine Einfach- oder Doppelschicht aus Siliziumoxid und/oder Siliziumnitrid sein kann. Diese Passivierungsschicht 3 sorgt dafür, daß die Leiterschicht 1 elektrisch isoliert, mechanisch geschützt und in einem konstanten Abstand zu der äußeren Oberfläche des Bauelementes gehalten wird. Um die Oberseite der Passivierungsschicht 3 vor Kratzern und mechanischem Abrieb zu schützen, ist erfindungsgemäß die Gleitschicht 4 aufgebracht, deren Oberseite beispielsweise bei einem Fingerabdrucksensor die Auflagefläche 5 für eine Fingerbire darstellt.

Als Material für die Gleitschicht kommen vorzugsweise Fette, Öle, grenz-/oberflächenaktive Substanzen (Tenside) und/oder Wachse bevorzugt infrage. Soll die Gleitschicht vor dem Einbau des Bauelementes in ein Gehäuse aufgebracht werden, muß wegen der höheren Temperaturbeanspruchung in der Regel auf temperaturbeständigere Materialien für die Gleitschicht zurückgegriffen werden. Das können unter anderem synthetische Öle oder Fette (zum Beispiel Silikonöl) oder synthetische Wachse (zum Beispiel Teflonwachs) sein. In der Stoffgruppe der Tenside zeigen Perfluorpolyether sehr gute mechanische Stabilität und chemische Resistenz. In einem besonders bevorzugten Ausführungsbeispiel der erfindungsgemäßen Gleitschicht wird diese gebildet durch eine Emulsion aus: Wasser, Paraffinöl, Propylenglykol, Stearinsäure, Palmitinsäure, TEA (Triethylamin), Bienenwachs, Carbormer 954, Methylparaben und Propylparaben. Parfum kann bedenkenlos zugesetzt werden.

## Patentansprüche

1. Halbleiterbauelement mit einer Oberflächenbeschichtung, die als Gleitschicht aus einer Kombination von Materialien aus der Gruppe von Fetten, Ölen, Tensiden und Wachsen gebildet wird und vor Kratzern und mechanischem Abrieb schützt, wobei eine Oberseite der Gleitschicht eine für eine Fingerbeere vorgesehene Auflagefläche eines Fingerabdrucksensors bildet.

2. Halbleiterbauelement nach Anspruch 1,
bei dem die Gleitschicht ein Silikonöl enthält.

3. Halbleiterbauelement nach Anspruch 1 oder 2,
bei dem die Gleitschicht einen Perfluorpolyether enthält.

4. Halbleiterbauelement mit einer Oberflächenbeschichtung, die als Gleitschicht eine Emulsion aus Wasser, Paraffinöl, Propylenglykol, Stearinsäure, Palmitinsäure, TEA, Bienenwachs, Carbomer 954, Methylparaben und Propylparaben ist.

5. Halbleiterbauelement nach Anspruch 4,
bei dem die Gleitschicht eine Oberseite einer Passivierungsschicht, die Polyimid ist oder eine Siliziumoxidschicht, eine Siliziumnitridschicht oder eine Siliziumoxidschicht und eine Siliziumnitridschicht umfaßt, vor Kratzern und mechanischem Abrieb schützt und
bei dem die Oberseite der Gleitschicht eine für eine Fingerbeere vorgesehene Auflagefläche eines Fingerabdrucksensors bildet.

## Claims

1. Semiconductor component with a surface coating which is formed as a sliding layer from a combination of materials from the group of fats, oils, surfactants and waxes and provides protection against scratches and mechanical abrasion, an upper side of the sliding layer forming a supporting surface of a fingerprint sensor provided for a finger pad.

2. Semiconductor component according to Claim 1, in which the sliding layer contains a silicone oil.

3. Semiconductor component according to Claim 1 or 2, in which the sliding layer contains a perfluoropolyether.

4. Semiconductor component with a surface coating which, as a sliding layer, is an emulsion comprising water, paraffin oil, propylene glycol, stearic acid, palmitic acid, TEA, beeswax, carbormer 954, methylparaben and propylparaben.

5. Semiconductor component according to Claim 4, in which the sliding layer provides protection against scratches and mechanical abrasion for an upper side of a passivation layer, which is polyimide or comprises a silicon oxide layer, a silicon nitride layer or a silicon oxide layer and a silicon nitride layer, and in which the upper side of the sliding layer forms a supporting surface of a fingerprint sensor provided for a finger pad.

## Revendications

1. Composant à semiconducteur ayant un revêtement de surface qui est formé d'une couche de glissement constituée d'une combinaison de matière choisie dans le groupe des graisses, des huiles, des agents tensioactifs et des cires, et qui protège des éraflures et de l'abrasion mécanique, une face supérieure de la couche de glissement formant une surface d'appui d'un capteur d'empreintes digitales prévu pour une face inférieure de la pulpe du doigt.

2. Composant à semiconducteur suivant la revendication 1, dans lequel la couche de glissement contient une huile de silicone.

3. Composant à semiconducteur suivant la revendication 1 ou 2, dans lequel la couche de glissement contient un perfluoropolyétheroxyde.

4. Composant à semiconducteur ayant un revêtement de surface qui, sous la forme d'une couche de glissement est une émulsion d'eau, d'huile de paraffine, de propylèneglycol, d'acide stéarique, d'acide palmitique, de TEA, de cire d'abeilles, de Carbomer 954, de méthylparaben et de propylparaben.

5. Composant à semiconducteur suivant la revendication 4, dans lequel la couche de glissement protège une face supérieure d'une couche de passivation qui est en polyimide ou comprend une couche d'oxyde de silicium, une couche de nitrure de silicium ou une couche d'oxyde de silicium et une couche de nitrure de silicium, protège des éraflures et de l'abrasion mécanique, et
dans lequel la face supérieure de la couche de glissement forme une surface d'appui d'un capteur d'empreintes digitales prévu pour une face inférieure de la pulpe du doigt.
